# EUROPEAN PATENT SPECIFICATION

(11) **EP 2 838 536 B1**
(45) Date of publication and mention of the grant of the patent: **09.08.2017**
(21) Application number: 13719340.5
(22) Date of filing: 18.04.2013
(51) Int. Cl.: A61K 31/485, A61K 9/20, A61K 45/06

(54) **IMMEDIATE RELEASE PHARMACEUTICAL COMPOSITIONS WITH ABUSE DETERRENT PROPERTIES**
PHARMAZEUTISCHE ZUSAMMENSETZUNGEN MIT UNMITTELBARER FREISETZUNG UND MISSBRAUCHSSICHEREN EIGENSCHAFTEN
COMPOSITIONS PHARMACEUTIQUES À LIBÉRATION IMMÉDIATE PRÉSENTANT DES PROPRIÉTÉS DE DISSUASION D'ABUS

(30) Priority: 18.04.2012 US 201261625718 P
(43) Date of publication of application: 25.02.2015
(73) Proprietor: Mallinckrodt LLC, Hazelwood, MO 63042 (US)
(72) Inventor: DIEZI, Thomas, A., Webster Groves, Missouri 63119 (US); RAMAN, Siva, N., Hazelwood, MO 63042 (US); PARK, Jae Han, Olivette, Missouri 63132 (US)
(74) Representative: Jones, Nicholas Andrew
(86) International application number: PCT/US2013/037046
(87) International publication number: WO 2013/158810

(56) References cited:
- EP-A1- 2 123 274
- WO-A1-2011/079074
- US-A1- 2008 102 121
- US-A1- 2011 054 038
- BEER BEATE ET AL: "[Impact of slow-release oral morphine on drug abusing habits in Austria]", NEUROPSYCHIATRIE, DUSTRI VERLAG, DEISENHOFEN, DE, vol. 24, no. 2, 1 January 2010 (2010-01-01), pages 108-117, XP009170205, ISSN: 0948-6259

## Description

### FIELD OF THE INVENTION

The present disclosure generally relates to pharmaceutical compositions that provide immediate release of active ingredients and have abuse deterrent properties.

### BACKGROUND OF THE INVENTION

Abuse of prescription drugs (particularly opioids) has become a serious societal problem. Such abuse places an enormous economic burden on society due to increased health care, work place, and criminal justice costs. Several routes of administration are commonly attempted by abusers. For example, the oral solid dosage form may be crushed or pulverized into a powder and administered intranasally (i.e., snorted) or dissolved in a suitable solvent (e.g., water) and administered parenterally (i.e., injected intravenously).

Attempts have been made to diminish the abuse of opioid solid dosage forms. One approach has been to include in the dosage form an opioid antagonist that is not orally active but will substantially block the analgesic effects of the opioid if one attempts to dissolve the opioid and administer it parenterally. Another approach has been to include gel-forming high molecular weight polymers that confer plasticity to the dosage form rendering them difficult to crush and pulverize into a powder. These high molecular weight polymers, however, retard the release of the active ingredient from the dosage forms, making them unsuitable for immediate release formulations.

Thus, there is a need for oral solid dosage forms that provide immediate release of the active ingredient yet are resistant to abuse.

A relevant prior art document is WO 2011/079074 A1.

### SUMMARY OF THE INVENTION

The present disclosure, therefore, provides a pharmaceutical composition comprising at least one active pharmaceutical ingredient (API) having a potential for abuse or a pharmaceutically acceptable salt thereof, at least one low molecular weight water-soluble polymer having a molecular weight of no more than 300,000 daltons, at least one polyglycol, at least one polysaccharide, at least one clay mineral, and, optionally, an effervescent system, wherein the pharmaceutical composition is an oral solid dosage form.

Another aspect of the present disclosure provides a process for preparing an oral solid dosage form, The process comprises forming a mixture comprising at least one active pharmaceutical ingredient (API) having a potential for abuse or a pharmaceutically acceptable salt thereof, at least one low molecular weight water-soluble polymer having a molecular weight of no more than 300,000 daltons, at least one polyglycol, at least one polysaccharide, at least one clay mineral, and, optionally, an effervescent system. The process further comprises forming the mixture into a solid dosage unit, and heating the solid dosage unit to yield the solid dosage form.

Other aspects and iterations of the disclosure are described in more detail below.

### REFERENCE TO COLOR FIGURES

The patent or application file contains at least one drawing executed in color. Copies of this patent or patent application publication with color drawings will be provided by the Office upon request and payment of the necessary fee.

### BRIEF DESCRIPTION OF THE FIGURES

**FIG. 1** presents SEM images of L-(+)-tartaric acid particles **(A);** L-(+)-tartaric acid particles coated with Pluronic F127 **(B);** and Pluronic F127-coated L-(+)-tartaric acid particles blended with talc **(C).**
**FIG. 2** shows the surface of a Pluronic F127-coated tartaric acid particle blended with talc. Elemental mapping shows that the majority of the surface is covered with talc, with limited Pluronic F127-coated surface visible. The carbon element of Pluronic F127 is denoted in red in panel **(A)** or purple in panel **(B).**

### DETAILED DESCRIPTION OF THE INVENTION

The present disclosure provides pharmaceutical compositions and processes for making oral solid dosage pharmaceutical compositions that provide rapid release of the active ingredients and have abuse deterrent properties. In addition to one or more pharmaceutically active ingredients having a potential for abuse, the compositions disclosed herein comprise at least one low molecular weight water-soluble polymer having a molecular weight of no more than 300,000 daltons, at least one polyglycol, at least one polysaccharide, at least one clay mineral, and an optional effervescent system. It was discovered that the combination of excipients comprising the compositions disclosed herein provides immediate release of the active ingredient(s) and makes the compositions resistant to crushing into fine powders and/or extracting with suitable solvents.

### (l) Pharmaceutical Compositions

One aspect of the present disclosure provides immediate release oral pharmaceutical compositions with abuse deterrent properties.

### (a) Components of the Compositions

The pharmaceutical compositions disclosed herein comprise at least one pharmaceutically active ingredient (API) having potential for abuse or a pharmaceutically acceptable salt thereof and a blend of pharmaceutically acceptable excipients. The excipients comprise low molecular weight water-soluble polymers having a molecular weight of no more than 300,000 daltons, polyglycols, polysaccharides, clay minerals, and, optionally, effervescent systems. It is the combination of these excipients that provides the immediate release and abuse deterrent properties of the compositions.

### (i) Low molecular weight water-soluble polymers

The pharmaceutical compositions comprise at least one low molecular weight water-soluble polymer having a molecular weight of no more than 300,000 daltons. The term "water-soluble polymer," as used herein, refers to polymers that are soluble or swellable in aqueous solutions. Some water-soluble polymers, however, may also be soluble in nonaqueous solvents such as the lower alcohols (e.g., methanol or ethanol).

The water-soluble polymer having a molecular weight of no more than 300,000 daltons may be synthetic, semi-synthetic, or naturally-occurring. Non-limiting examples of suitable water-soluble polymers include polyalkylene oxides such as polyethylene oxides (PEO); cellulose ethers such as hydroxypropyl cellulose, methyl cellulose, hydroxymethyl cellulose, hydroxyethyl cellulose, hydraxypropylmethyl cellulose, carboxymethyl cellulose, and the like; polyolefinic alcohols such as polyvinyl alcohols: polyvinyl lactams such as polyvinylpyrrolidone, polvinyl caprolactam, and the like; and polycarboxylic acids, such as polyacrylic acids.

As used herein, the term "low molecular weight polymer" refers to a polymer having a molecular weight of no more than 300,000 Da. In various embodiments, the approximate molecular weight of the low molecular weight polymer may range from 300,000 to about 250,000 Da, from about 250,000 to about 200,000 Da, from about 200,000 to about 150,000 Da, from about 150,000 to about 100,000 Da, from 100,000 to about 75,000 Da, from about 75,000 to about 50,000 Da, from about 50,000 to about 25,000 Da, or from about 25,000 to about 1000 Da.

In certain embodiments, the low molecular weight water-soluble polymer may be a polyethylene oxide. In an exemplary embodiment, the polyethylene oxide may have an approximate molecular weight of about 100,000 Da.

The amount of low molecular weight water-soluble polymer present in the composition can and will vary depending upon the desired properties of the composition, as well as the identity and amounts of other components present in the composition. In general, the amount of low molecular weight polymer present in the composition may range from about 5% to about 60% by weight of the composition. In various embodiments, the amount of low molecular weight polymer present in the composition may range from about 5% to about 15%, from about 15% to about 25%, from about 25% to about 40%, or from about 40% to about 60% by weight of the total weight of the composition. In exemplary embodiments, the amount of low molecular weight polymer present in the composition may range from about 20% to about 50% by weight of the total weight of the composition.

### (ii) Polyglycol

The pharmaceutical compositions also comprise at least one polyglycol. Suitable polyglycols have wax-like properties in the solid state and emulsification properties in the liquid state. Thus, the presence of the polyglycol in the compositions may provide stickiness to solid dosage forms such that they are difficult to crush into fine powders. Additionally, the polyglycol may facilitate the suspension of clay mineral particles in mixtures formed when the pharmaceutical compositions disclosed here are mixed with small volumes of suitable solvents.

Non-limiting examples of suitable polyglycols include copolymers of ethylene glycol and propylene glycol, polyethylene glycols, polypropylene glycols, polyethylene-polypropylene glycols, polyoxyethylene alkyl esters, polyoxyethylene sorbitan fatty acid esters, polyoxyethylene stearates, and combinations thereof. In one embodiment, the polyglycol may be a copolymer of ethylene glycol and propylene glycol. Such copolymers are also known as poloxamers and are available under the tradenames PLURONIC^{®} or KOLLIPHOR™. For example, the polyglycol may be PLURONIC^{®} F38, F68, F77, F87, F88, F98, F108, F127, or combinations thereof. Other than polyglycols, self-emulsifying waxes may also be used. An example of a suitable self-emulsifying wax is Emulsifying Wax NF, which is a blend of cetearyl alcohol, polysorbate 60, PEG-150 stearate, and steareth-20 (which is a synthetic polymer composed of PEG and stearyl alcohol).

In exemplary embodiments, the polyglycol may be PLURONIC^{®} F127 (which is also known as poloxamer 407 or KOLLIPHOR™ P407). In another preferred embodiment, the polyglycol may be PLURONIC^{®} F68 (which is also known as poloxamer 188 or KOLLIPHOR™ P188). In one exemplary embodiment, the polyglycol may comprise a combination of PLURONIC^{®} F68 and PLURONIC^{®} F127.

The amount of the polyglycol present in the composition can and will vary depending upon the desired properties of the composition. In general, the amount of the polyglycol present in the composition may range from about 5% to about 50% by weight of the composition. In various embodiments, the amount of the polyglycol present in the composition may range from about 5% to about 10%, from about 10% to about 20%, from about 20% to about 30%, from about 30% to about 40%, or from about 40% to about 50% by weight of the total weight of the composition. In exemplary embodiments, the amount of the polyglycol present in the composition may range from about 10% to about 45% by weight of the composition.

### (iii) Polysaccharide

The pharmaceutical compositions disclosed herein also comprise at least one polysaccharide. In general, the polysaccharide functions as an emulsifying agent. For example, the polysaccharide may facilitate the suspension of clay mineral particles in dispersions formed when the pharmaceutical composition disclosed herein is mixed with a small volume of a suitable solvent. A variety of polysaccharides may be included in the pharmaceutical compositions. Suitable polysaccharides include, without limit, celluloses, starches, pectins, chitins, gums (i.e., polysaccharides derived from plants or microbes), combinations thereof, and derivatives thereof. In exemplary embodiments, the polysaccharide may be a gum. Non-limiting examples of suitable gums include xanthan gum, acacia gum, diutan gum, gellan gum, guar gum, fenugreek gum, locust bean gum, pullulan, welan gum, or combinations thereof. In exemplary embodiments, the polysaccharide may be xanthan gum.

The mesh size of the polysaccharide may can and will vary. For example, the mesh size may range from coarse (e.g., 45 mesh) to fine (e.g., 200 mesh). In one embodiment, the polysaccharide may be xanthan gum having a mesh size of about 60.

The amount of the polysaccharide in the composition can and will vary depending upon the desired properties of the composition. In general, the amount of polysaccharide present in the composition may range from about 1% to about 10% by weight of the total weight of the composition. In various embodiments, the amount of polysaccharide present in the composition may range from about 1 % to about 3%, from about 3% to about 6%, or from about 6% to about 10% by weight of the total weight of the composition. In exemplary embodiments, the amount of polysaccharide present in the composition may range from about 2% to about 6% by weight of the total weight of the composition.

### (iv) Clay mineral

The pharmaceutical compositions disclosed herein also comprise at least one clay mineral. As used herein, a "clay mineral" refers to a hydrated aluminum phyllosilicate or a hydrated magnesium silicate. In general, clay minerals comprise small insoluble particles. Additionally, some clay minerals are absorbent and swell when wet. For example, sodium bentonite can absorb water several times its dry weight and swell to approximately 5-10 times its own volume. Thus, mixing a clay mineral with water or another solvent forms a colloidal dispersion, i.e., the clay mineral particles are visible and stay suspended for extended periods of time.

A variety of clay minerals are suitable for inclusion in the pharmaceutical composition. Non-limiting examples of suitable clay minerals include bentonites (e.g., sodium bentonite, calcium bentonite, potassium bentonite), kaolinites, nontronites, montmorillonites, pyrophyllites, saponites, sauconites, vermiculites, talc, and combinations thereof. In one exemplary embodiment, the clay mineral may be a combination of sodium bentonite and talc. The talc may be micronized. In another exemplary embodiment, the clay mineral may be sodium bentonite.

The amount of the clay mineral present in the composition can and will vary depending upon the desired properties of the composition. In general, the amount of the clay mineral present in the composition may range from about 1% to about 30% by weight of the total weight of the composition. In various embodiments, the amount of the clay mineral present in the composition may range from about 1% to about 5%, from about 5% to about 10%, from about 10% to about 20%, or from about 20% to about 30% by weight of the composition. In exemplary embodiments, the amount of clay mineral present in the composition may range from about 5% to about 25% by weight of the composition.

### (v) Optional effervescent system

The pharmaceutical compositions disclosed herein may optionally comprise an effervescent system. As used herein, an "effervescent system" refers to a system generally comprising an acid component and a base component, wherein the system liberates carbon dioxide upon contact with an aqueous solution. Without being bound by any particular theory, it is believed that the effervescent system facilitates rapid dissolution of an active ingredient from the composition.

The acid component of the effervescent system may be an organic acid, an inorganic acid, or combinations thereof. Non-limiting examples of suitable acids include adipic acid, ascorbic acid, benzoic acid, citric acid, fumaric acid, glutaric acid, lactic acid, lauric acid, malic acid, maleic acid, malonic acid, oxalic acid, phthalic acid, sorbic acid, succinic acid, tartaric acid, ammonium phosphate, potassium bitartrate, potassium phosphate, dipotassium phosphate, disodium pyrophosphate, sodium acid pyrophosphate, sodium phosphate, disodium phosphate, and combinations thereof. In one exemplary embodiment, the acid component of the effervescent system may be a combination of citric acid and tartaric acid. In another exemplary embodiment, the acid component of the effervescent system may be tartaric acid.

The base component of the effervescent system may be a bicarbonate, a carbonate, or a combination thereof. In various embodiments, the base may be an alkali metal bicarbonate, an alkaline earth metal bicarbonate, an alkali metal carbonate, an organic carbonate, or combinations thereof. Non-limiting examples of suitable bases include ammonium bicarbonate, calcium bicarbonate, lithium bicarbonate, magnesium bicarbonate, potassium bicarbonate, sodium bicarbonate, arginine carbonate, ammonium carbonate, calcium carbonate, lysine carbonate, potassium magnesium carbonate, sodium carbonate, sodium glycine carbonate, sodium sesquicarbonate, zinc carbonate, and combinations thereof. In exemplary embodiments, the base component of the effervescent system may be an alkali metal bicarbonate (such as sodium bicarbonate or potassium bicarbonate), an alkali metal carbonate (such as sodium carbonate or potassium carbonate), or combinations thereof. In one exemplary embodiment, the base component may be sodium bicarbonate. In another exemplary embodiment, the base component may be heat-treated sodium bicarbonate (for example EfferSoda^{®}12).

The amount of the effervescent system present in the composition can and will vary depending upon the desired properties of the composition. In general, in embodiments in which the effervescent system is present, the amount of the effervescent system may range from about 5% to about 70% by weight of the composition. For example, the amount of the effervescent system present in the composition may range from about 5% to about 10%, from about 10% to about 20%, from about 20% to about 30%, from about 30% to about 40%, from about 40% to about 50%, from about 50% to about 60%, or from about 60% to about 70% by weight of the composition. In exemplary embodiments in which the pharmaceutical composition comprises the effervescent system, the amount of the effervescent system present in the composition may range from about 15% to about 50% by weight of the composition.

The mole to mole ratio of the acid component to the base component in the effervescent system may also vary depending upon the identity of the acid and base components. In general, the mole to mole ratio of the acid component to the base component in the effervescent system may range from about 1:3 to about 3:1. For example, the mole to mole ratio of the acid component to the base component in the effervescent system may be about 1:3, 1:2, 1:1, 2:1, 3:1, or any ratio in-between. In am exemplary embodiment, the mole to mole ratio of the acid component to the base component in the effervescent system may be about 1:2.

### (vi) APIs

The pharmaceutical compositions disclosed herein comprise APIs having a potential for abuse. A list of APIs include opioid analgesic agents (e.g., adulmine, alfentanil, allocryptopine, allylprodine, alphaprodine, anileridine, aporphine, benzylmorphine, berberine, bicuculine, bicucine, bezitramide, buprenorphine, bulbocaprine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, fentanyl, heroin, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propoxyphene, sufentanil, tapentadol, tilidine, and tramadol); non-opioid analgesic agents (e.g., acetylsalicylic acid, acetaminophen, paracetamol, ibuprofen, ketoprofen, indomethacin, diflunisol, naproxen, ketorolac, dichlophenac, tolmetin, sulindac, phenacetin, piroxicam, and mefamanic acid); anti-inflammatory agents (e.g., glucocorticoids such as alclometasone, fluocinonide, methylprednisolone, triamcinolone and dexamethasone; non-steroicfal anti-inflammatory agents such as celecoxib, deracoxib, ketoprofen, lumiracoxib, meloxicam, parecoxib, rofecoxib, and valdecoxib); antitussive agents (e.g., dextromethorphan, codeine, hydrocodone, caramiphen, carbetapentane, and dextromethorphan); antipyretic agents (e.g., acetylsalicylic acid and acetaminophen); antibiotic agents (e.g., aminoglycosides such as, amikacin, gentamicin, kanamycin, neomycin, netilmicin, streptomycin, and tobramycin; carbecephem such as loracarbef; carbapenems such as certapenem, imipenem, and meropenem; cephalosporins such as cefadroxil cefazolin, cephalexin, cefaclor, cefamandole, cephalexin, cefoxitin, cefprozil, cefuroxime, cefixime, cefdinir, cefditoren, cefoperazone, cefotaxime, cefpodoxime, ceftazidime, ceftibuten, ceftizoxime, and ceftriaxone; macrolides such as azithromycin, clarithromycin, dirithromycin, erythromycin, and troleandomycin; monobactam; penicillins such as amoxicillin, ampicillin, carbenicillin, cloxacillin, dicloxacillin, nafcillin, oxacillin, penicillin G, penicillin V, piperacillin, and ticarcillin; polypeptides such as bacitracin, colistin, and polymyxin B; quinolones such as ciprofloxacin, enoxacin, gatifloxacin, levofloxacin, lomefloxacin, moxifloxacin, norfloxacin, ofloxacin, and trovafloxacin; sulfonamides such as mafenide, sulfacetamide, sulfamethizole, sulfasalazine, sulfisoxazole, and trimethoprim-sulfamethoxazole; tetracyclines such as demeclocycline, doxycycline, minocycline, and oxytetracycline); antimicrobial agents (e.g., ketoconazole, amoxicillin, cephalexin, miconazole, econazole, acyclovir, and nelfinavir); antiviral agents (e.g., acyclovir, gangciclovir, oseltamivir, and relenza); steroids (e.g., estradiol, testosterone, cortisol, aldosterone, prednisone, and cortisone); amphetamine stimulant agents (e.g., amphetamine and amphetamine-like drugs); non-amphetamine stimulant agents (e.g., methylphenidate, nicotine, and caffeine); laxative agents (e.g., bisacodyl, casanthranol, senna, and castor oil); anti-nausea agents (e.g., dolasetron, granisetron, ondansetron, tropisetron, meclizine, and cyclizine); anorexic agents (e.g., fenfluramine, dexfenfluramine, mazindol, phentermine, and aminorex); antihistaminic agents (e.g., phencarol, cetirizine, cinnarizine, ethamidindole, azatadine, brompheniramine, hydroxyzine, and chlorpheniramine); antiasthmatic agents (e.g., zileuton, montelukast, omalizumab, fluticasone, and zafirlukast); antidiuretic agents (e.g., desmopressin, vasopressin, and lypressin); an antiflatulant agent (e.g., simethicone); antimigraine agents (e.g., naratriptan, frovatriptan, eletriptan, dihydroergotamine, zolmitriptan, almotriptan, and sumatriptan); antispasmodic agents (e.g., dicyclomine, hyoscyamine, and peppermint oil); antidiabetic agents (e.g., methformin, acarbose, miglitol, pioglitazone, rosiglitazone, troglitazone, nateglinide, repaglinide, mitiglinide, saxagliptin, sitagliptine, vildagliptin, acetohexamide, chlorpropamide, gliclazide, glimepiride, glipizide, glyburide, tolazamide, and tolbutamide); respiratory agents (e.g., albuterol, ephedrine, metaproterenol, and terbutaline); sympathomimetic agents (e.g., pseudoephedrine, phenylephrine, phenylpropanolamine, epinephrine, norepinephrine, dopamine, and ephedrine); H2 blocking agents (e.g., cimetidine, famotidine, nizatidine, and ranitidine); antihyperlipidemic agents (e.g., clofibrate, cholestyramine, colestipol, fluvastatin, atorvastatin, genfibrozil, lovastatin, niacin, pravastatin, fenofibrate, colesevelam, and simvastatin); antihypercholesterol agents (e.g., lovastatin, simvastatin, pravastatin, fluvastatin, atorvastatin, cholestyramine, colestipol, colesevelam, nicotinic acid, gemfibrozil, and ezetimibe); cardiotonic agents (e.g., digitalis, ubidecarenone, and dopamine); vasodilating agents (e.g., nitroglycerin, captopril, dihydralazine, diltiazem, and isosorbide dinitrate); vasoconstricting agents (e.g., dihydroergotoxine and dihydroergotamine); anticoagulants (e.g., warfarin, heparin, and Factor Xa inhibitors); sedative agents (e.g., amobarbital, pentobarbital, secobarbital, clomethiazole, diphenhydramine hydrochloride, and alprazolam); hypnotic agents (e.g., zalepion, zolpidem, eszopiclone, zopiclone, chloral hydrate, and clomethiazole); anticonvulsant agents (e.g., lamitrogene, oxycarbamezine, phenytoin, mephenytoin, ethosuximide, methsuccimide, carbamazepine, valproic acid, gabapentin, topiramate, felbamate, and phenobarbital); muscle relaxing agents (e.g., baclofen, carisoprodol, chlorzoxazone, cyclobenzaprine, dantrolene sodium, metaxalone, orphenadrine, pancuronium bromide, and tizanidine); antipsychotic agents (e.g., phenothiazine, chlorpromazine, fluphenazine, perphenazine, prochlorperazine, thioridazine, trifluoperazine, haloperidol, droperidol, pimozide, clozapine, olanzapine, risperidone, quetiapine, ziprasidone, melperone, and paliperidone); antianxiolitic agents (e.g., lorazepam, alprazolam, clonazepam, diazepam, buspirone, meprobamate, and flunitrazepam); antihyperactive agents (e.g., methylphenidate, amphetamine, and dextroamphetamine); antihypertensive agents (e.g., alpha-methyldopa, chlortalidone, reserpine, syrosingopine, rescinnamine, prazosin, phentolamine, felodipine, propanolol, pindolol, labetalol, clonidine, captopril, enalapril, and lisonopril); anti-neoplasia agents (e.g., taxol, actinomycin, bleomycin A2, mitomycin C, daunorubicin, doxorubicin, epirubicin, idarubicin, and mitoxantrone); soporific agents (e.g., zolpidem tartrate, eszopiclone, ramelteon, and zaleplon); tranquilizer agents (e.g., alprazolam, clonazepam, diazepam, flunitrazepam, lorazepam, triazolam, chlorpromazine, fluphenazine, haloperidol, loxapine succinate, perphenazine, prochlorperazine, thiothixene, and trifluoperazine); decongestant agents (e.g., ephedrine, phenylephrine, naphazoline, and tetrahydrozoline); beta blockers (e.g., levobunolol, pindolol, timolol maleate, bisoprolol, carvedilol, and butoxamine); alpha blockers (e.g., doxazosin, prazosin, phenoxybenzamine, phentolamine, tamsulosin, alfuzosin, and terazosin); non-steroidal hormones (e.g., corticotropin, vasopressin, oxytocin, insulin, oxendolone, thyroid hormone, and adrenal hormone); erectile disfunction improvement agents; herbal agents (e.g., glycyrrhiza, aloe, garlic, nigella sativa, rauwolfia, St John's wort, and valerian); enzymes (e.g., lipase, protease, amylase, lactase, lysozyme, and urokinase); humoral agents (e.g., prostaglandins, natural and synthetic, for example, PGE1, PGE2alpha, PGF2alpha, and the PGE1 analog misoprostol); psychic energizers (e.g., 3-(2-aminopropy)indole and 3-(2-aminobutyl)indole); nutritional agents; essential fatty acids; non-essential fatty acids; vitamins; minerals; and combinations thereof.

Only APIs having a potential for abuse of those mentioned above may be incorporated in the composition described herein in any suitable form, such as, for example, as a pharmaceutically acceptable salt, uncharged or charged molecule, molecular complex, solvate or anhydrate, and, if relevant, isomer, enantiomer, racemic mixture, and/or mixtures thereof. Furthermore, the API having a potential for abuse may be in any of its crystalline, semicrystalline, amorphous, or polymorphous forms.

The API present in the composition has a potential for abuse. For example, the API may be an opioid analgesic agent, a stimulant agent, a sedative agent, a hypnotic agent, an antianxiolitic agent, or a muscle relaxing agent.

In another embodiment, the API present in the composition may be a combination of an opioid analgesic and a non-opioid analgesic. Suitable opioid and non-opioid analgesics are listed above.

In exemplary embodiments, the API in the composition may be an opioid analgesic. Preferred opioid analgesics include oxycodone, oxymorphone, hydrocodone, hydromorphone, codeine, and morphine.

The amount of API(s) in the composition can and will vary depending upon the active agent. In embodiments in which the API is an opioid analgesic, the amount of opioid in the composition may range from about 2 mg to about 160 mg. In various embodiments, the amount of opioid in the composition may range from about 2 mg to about 10 mg, from about 10 mg to about 40 mg, from about 40 mg to about 80 mg, or from about 80 mg to about 160 mg. In certain embodiments, the amount of opioid in the composition may be about 5 mg, 7.5 mg, 10 mg, 12.5 mg, 15 mg, 17.5 mg, 20 mg, 22.5 mg, 25 mg, 27.5 mg, 30 mg, 32.5 mg, 35 mg, 37.5 mg, 40 mg, 45 mg, 50 mg, 60 mg, 70 mg, 80 mg, 100 mg, 120 mg, 140 mg, or 160 mg.

In embodiments in which the opioid is oxycodone hydrochloride, the total amount of oxycodone hydrochloride present in the pharmaceutical composition may range from about 2 mg to about 80 mg. In certain embodiments, the amount of oxycodone hydrochloride in the pharmaceutical composition may range from about 2 mg to about 10 mg, from about 10 mg to about 30 mg, or from about 30 mg to about 80 mg. In exemplary embodiments, the amount of oxycodone hydrochloride present in the pharmaceutical composition may be about 5 mg, about 10 mg, about 15 mg, about 20 mg, about 30 mg, about 40 mg, about 60 mg, or about 80 mg.

In embodiments in which the opioid is oxymorphone hydrochloride, the total amount of oxymorphone hydrochloride present in the pharmaceutical composition may range from about 2 mg to about 80 mg. In certain embodiments, the amount of oxymorphone hydrochloride present in the pharmaceutical composition may range from about 2 mg to about 10 mg, from about 10 mg to about 30 mg, or from about 30 mg to about 80 mg. In exemplary embodiments, the amount of oxymorphone hydrochloride present in the pharmaceutical composition may be about 5 mg, about 10 mg, about 20 mg, about 30 mg, about 40 mg, about 60 mg, or about 80 mg.

### (vii) Optional excipients

In various embodiments, the pharmaceutical composition disclosed herein may further comprise at least one additional pharmaceutically acceptable excipient. Non-limiting examples of suitable excipients include lubricants, disintegrants, binders, fillers, diluents, antioxidants, chelating agents, flavoring agents, coloring agents, taste masking agents, and combinations thereof.

In one embodiment, the excipient may be a lubricant. Non-limiting examples of suitable lubricants include magnesium stearate, calcium stearate, zinc stearate, hydrogenated vegetable oils, sterotex, polyoxyethylene monostearate, polyethylene glycol, sodium stearyl fumarate, sodium benzoate, sodium lauryl sulfate, magnesium lauryl sulfate, light mineral oil, and combinations thereof. In a preferred embodiment, the composition may comprise magnesium stearate as a lubricant.

In another embodiment, the excipient may be a disintegrant or a superdisintegrant. The disintegrant or superdisintegrant may not affect disintegration of the composition in the typical sense, but may improve hardness of the solid dose compositions disclosed herein. Suitable disintegrants include, without limit, starches (such as corn starch, potato starch, and the like), pregelatinized and modified starches thereof, micro-crystalline cellulose, alginates, sodium starch glycolate, and gums (such as agar, guar, locust bean, karaya, pectin, and tragacanth). Non-limiting examples of suitable superdisintegrants include crospovidine, croscarmellose sodium, sodium starch glycolate, low substituted hydroxypropyl cellulose, and combinations thereof. In one preferred embodiment, the composition may comprise crospovidine as a superdisintegrant. In another preferred embodiment, the composition may comprise F-MELT^{®}, a proprietary blend of carbohydrates, disintegrants, and inorganic ingredients, as a superdisintegrant.

In a further embodiment, the excipient may be a binder. Suitable binders include, but are not limited to, starches, pregelatinized starches, gelatin, polyvinylpyrolidone, cellulose, methylcellulose, sodium carboxymethylcellulose, ethylcellulose, polyacrylamides, polyvinyloxoazolidone, polyvinylalcohols, C12-C18 fatty acid alcohol, polyethylene glycol, polyols, saccharides, oligosaccharides, polypeptides, peptides, and combinations thereof.

In another embodiment, the excipient may be a filler. Suitable fillers include carbohydrates, inorganic compounds, and polyvinylpyrrolidone. By way of non-limiting examples, the filler may be calcium sulfate, calcium phosphate, calcium silicate, microcrystalline cellulose, starch, modified starches, lactose, sucrose, mannitol, sorbitol, or combinations thereof.

In another embodiment, the excipient may include a diluent. Non-limiting examples of diluents suitable for use include pharmaceutically acceptable saccharides such as sucrose, dextrose, lactose, microcrystalline cellulose, fructose, xylitol, and sorbitol; polyhydric alcohols; starches; premanufactured direct compression diluents; and mixtures of any of the foregoing.

In yet another embodiment, the excipient may be an antioxidant. Suitable antioxidants include, without limit, ascorbyl palmitate, butylated hydroxyanisole, a mixture of 2 and 3 tertiary-butyl-4-hydroxyanisole, butylated hydroxytoluene, sodium isoascorbate, dihydroguaretic acid, potassium sorbate, sodium bisulfate, sodium metabisulfate, sorbic acid, potassium ascorbate, vitamin E, 4-chloro-2,6-ditertiarybutylphenol, alphatocopherol, and propylgallate, and combinations thereof.

In an alternate embodiment, the excipient may be a chelating agent. Non-limiting examples of suitable chelating agents include ethylenediamine tetracetic acid (EDTA) and its salts, N-(hydroxy-ethyl)ethylenediaminetriacetic acid, nitrilotriacetic acid (NIA), ethylene-bis(oxyethylene-nitrilo}tetraacetic acid, 1,4,7,10-tetraazacyclodo-decane-N,N',N",N"'-tetraacetic acid, 1,4,7,10-tetraaza-cyclododecane-N,N',N"-triacetic acid, 1,4,7-tris(carboxymethyl)-10-(2'-hydroxypropyl)- 1,4,7,10-tetraazocyclodecane, 1,4,7-triazacyclonane-N,N',N"-triacetic acid, 1,4,8,11 -tetraazacyclotetra-decane-N,N',N",N"'-tetraacetic acid; diethylenetriamine-pentaacetic acid (DTPA), ethylenedicysteine, bis(aminoethanethiol)carboxylic acid, triethylenetetraamine-hexaacetic acid, 1,2-diaminocyclohexane-N,N,N',N'-tetraacetic acid, and combinations thereof.

In a further embodiment, the excipient may be a flavoring agent. Flavouring agents may be chosen from synthetic flavor oils and flavoring aromatics and/or natural oils, extracts from plants, leaves, flowers, fruits, and combinations thereof.

In still another embodiment, the excipient may be a coloring agent. Suitable color additives include food, drug and cosmetic colors (FD&C), drug and cosmetic colors (D&C), or external drug and cosmetic colors (Ext. D&C).

In yet another embodiment, the excipient may be a taste-masking agent. Taste-masking materials include cellulose hydroxypropyl ethers (HPC); low-substituted hydroxypropyl ethers (L-HPC); cellulose hydroxypropyl methyl ethers (HPMC); methylcellulose polymers and mixtures thereof; polyvinyl alcohol (PVA); hydroxyethylcelluloses; carboxymethylcelluloses and salts thereof; polyvinyl alcohol and polyethylene glycol copolymers; monoglycerides or triglycerides; polyethylene glycols; acrylic polymers; mixtures of acrylic polymers with cellulose ethers; cellulose acetate phthalate; and combinations thereof.

The amount of excipient(s) in the composition can and will vary depending upon the identity and amounts of the components of the composition and API(s) detailed above.

### (viii) Optional film coating

In other embodiments, the pharmaceutical composition may further comprise an optional film coating. Typically, the film coating comprises water-soluble polymer(s) and does not affect the immediate release or tamper resistant properties of the composition. The film coating may provide moisture protection, enhanced appearance, increased mechanical integrity, improved swallowability, improved taste, and/or masking of odors.

Film coatings are well known in the art, e.g., they are commercially available under the tradename OPADRY^{®}. Typically, a film coating comprises at least one water-soluble polymer and at least one plasticizer. Non-limiting examples of suitable polymers include hydroxypropyl methylcellulose, hydroxypropyl cellulose, hydroxypropyl ethylcellulose, ethylcellulose, methylcellulose, cellulose acetate phthalate, microcrystalline cellulose and carrageenan, acrylic polymers, polyvinyl alcohol, anionic and cationic polymers of methacrylic acid, copolymers of methacrylates, copolymers of acrylates and methacrylates, copolymers of ethacrylate and methylmethacrylate, polyvinylacetate phthalate, and shellac. Examples of suitable plasticizers include, without limit, triethyl citrate (TEC), acetyltriethyl citrate (ATEC), acetyl tri-n-butyl citrate (ATBC), dibutyl sebacate, diethyl phthalate, and triacetin. The film coating may optional comprise additional agents such as a coloring agent, a filler, a flavoring agent, a taste-masking agent, a surfactant, an anti-tacking agent, and/or an anti-foaming agent. Suitable examples of these agents are well known in the art and/or are detailed above.

### (ix) Exemplary embodiments

In one exemplary embodiment, the pharmaceutical composition may be a non-effervescent formulation in which the low molecular weight water-soluble polymer may be a polyethylene oxide having a molecular weight of about 100,000 Da; the polyglycol may be a poloxamer (e.g., Pluronic F68 and/or Pluronic F127); the polysaccharide may be xanthan gum; and the clay mineral may be sodium bentonite or a combination of sodium bentonite and talc. The composition may further comprise a superdisintegrant and a lubricant. The API may be an opioid chosen from oxycodone, oxymorphone, hydrocodone, hydromorphone, codeine, and morphine. The composition of a non-effervescent formulation is shown in the Table A below:

**Table A. Exemplary Non-Effervescent Formulation.**

| **Ingredient** | **% w/w Range** | **Function** |
|---|---|---|
| API | 5 - 20 | Active |
| PEO 100,000 g/mol | 20 - 35 | Physical Plasticity / Prevent pulverization |
| Pluronic | 10 - 45 | Emulsification / Prevent pulverization |
| Xanthan Gum | 2 - 6 | Emulsification |
| Na-Bentonite | 5 - 15 | Disintegrant /Visual deterrent |
| Talc | 0 - 10 | Disintegrant / visual deterrent |
| Superdisintegrant (e.g., F-melt) | 0 - 10 | Disintegrant / Provides tablet hardness |
| Magnesium Stearate | 1 | Lubricant |

In another exemplary embodiment, the pharmaceutical composition may be an effervescent formulation in which the low molecular weight water-soluble polymer may be a polyethylene oxide having a molecular weight of about 100,000 Da; the polyglycol may be a poloxamer (e.g., Pluronic F68 and/or Pluronic F127); the polysaccharide may be xanthan gum; the clay mineral may be sodium bentonite or a combination of sodium bentonite and talc; the base component of the effervescent system may comprise sodium bicarbonate or a combination of sodium bicarbonate and sodium carbonate; the acid component of the effervescent system may comprise tartaric acid and a combination of tartaric acid and citric acid. The composition may further comprise a lubricant. The API may be an opioid chosen from oxycodone, oxymorphone, hydrocodone, hydromorphone, codeine, and morphine. The composition of an effervescent formulation is shown in the Table B below:

**Table B. Exemplary Effervescent Formulation.**

| **Ingredient** | **% w/w Range** | **Function** |
|---|---|---|
| API | 5 - 20 | Active |
| PEO 100,000 g/mol | 30 - 50 | Physical Plasticity / Prevent pulverization |
| Pluronic | 10 - 30 | Emulsification / Prevent pulverization |
| Xanthan Gum | 2 - 6 | Emulsification |
| Na-Bentonite | 5 - 15 | Disintegrant / Visual deterrent |
| Talc | 0 - 10 | Disintegrant / Visual deterrent |
| Base Component | 10 - 20 | Effervescent component |
| Citric Acid Anhydrous | 0 - 15 | Effervescent component / Plasticizer |
| Tartaric Acid | 5 - 15 | Effervescent component |
| Magnesium Stearate | 1 | Lubricant |

### (b) Dosage Forms

The physical form of the pharmaceutical compositions disclosed herein can and will vary. In general, the pharmaceutical compositions are solid dosage forms for oral administration. Suitable solid dosage forms include tablets, caplets, granules, pills, and capsules. Such dosage forms may be prepared using conventional methods known to those in the field of pharmaceutical formulation and described in the pertinent texts, e.g., in Gennaro, A. R., editor. "Remington: The Science & Practice of Pharmacy", 21 st ed., Williams & Williams, and in the "Physician's Desk Reference", 2006, Thomson Healthcare.

In preferred embodiments, the solid dosage form may be a tablet. Non-limiting types of tablets include coated tablets, uncoated tablets, compressed tablets, compacted tablets, molded tablets, layered tablets, bilayer tablets, extruded tablets, multiparticle tablets, monolithic tablets, and matrix tablets. In exemplary embodiments, the pharmaceutical composition may be a solid dosage form comprising a tablet.

In general, the tablet has sufficient mechanical strength and/or resiliency that it is difficult to crush into a powder. The mechanical strength of the tablet may be quantified its hardness or crushing strength, friability, and/or tensile strength.

In preferred embodiments, the tablet may have a hardness or crushing strength of at least about 7 kilopond (kp). In various embodiments, the tablet may have a hardness or crushing strength that ranges from about 7 kp to about 10 kp, from about 10 kp to about 15 kp, from about 15 kp to about 20 kp, from about 20 kp to about 25 kp, or greater than 25 kp..

In general, the tablet has a friability of no greater than about 1.0%, or more preferably no greater than about 0.5%. In certain embodiments, the tablet may have a friability of less than about 1.0%, less than about 0.5%, less than about 0.3%, less than about 0.2%, less than about 0.1%, less than about 0.05%, or less than about 0.01%. In still another embodiment, the tablet may have a friability of zero.

### (c) In Vitro Release Properties of the Composition

The pharmaceutical composition disclosed herein is formulated such that the API in the composition is rapidly released. Thus, the composition is termed an immediate release composition. As used herein, "immediate release" generally refers to an average release of at least 70% of the API within 45 minutes.

The *in vitro* dissolution of the API from the composition disclosed herein may be measured using an USP-approved release procedure. For example, dissolution may be measured using an USP Type 2 paddle apparatus, at a paddle speed of 50 rpm or 100 rpm, and a constant temperature of 37 ± 0.5°C. The dissolution procedure may be performed in the presence of 500 mL, 900 mL, or 1,000 mL of a suitable dissolution medium (e.g., having a pH from 1.0 to 6.8). Non-limiting examples of suitable dissolution media include water, phosphate buffer (pH 6.8), acetate buffer (pH 4.5), and 0.1 N HCl.

The pharmaceutical compositions disclosed herein provide immediate release of the API. In some embodiments, the pharmaceutical composition may have an average release of about 50%, 55%, 60%, 65%, 70%, 75%, 80%, 85%, 90%, 95%, or 99% of the API within about 45 minutes. In other embodiments, the pharmaceutical composition may have an average release of about 50%, 55%, 60%, 65%, 70%, 75%, 80%, 85%, 90%, 95%, or 99% of the API within about 30 minutes.

### (d) Abuse Deterrent Properties of the Composition

The blend of components in the pharmaceutical compositions disclosed herein provides abuse deterrent or tamper resistant properties. For example, the disclosed compositions are resistant to crushing, grinding, cutting, or pulverizing to form fine powders. The combination of the low molecular weight water-soluble polymer and the polyglycol may contribute to the crush resistance of pharmaceutical compositions by imparting plastic properties to the solid dosage form. In some embodiments, the solid dosage compositions may form large waxy flakes when subjected to various crushing/pulverizing means (e.g., such as a pill crusher, a tablet grinder, a hammer, and/or trituration with a mortar and pestle). In exemplary embodiments, the solid dosage compositions are plastic and cannot be ground or pulverized into particles. When such compositions are subjected to milling (e.g., using a high-shear mill, a ball mill, a co-mill, pill crusher, a tablet grinder, a coffee grinder, a blender, a hammer, or another apparatus to reduce particle size) only large particles are formed.

In one embodiment, when the composition is milled for 9 minutes to form particles size, more than about 30%, 40%, 50%, 60%, 70%, 80%, 90%, or 95% of the particles have an average diameter of at least about 250 microns. In another embodiment, after 9 minutes of milling to form particles, more than about 30%, 40%, 50%, 60%, 70%, 80%, 90%, or 95% of the particles have an average diameter of at least about 500 microns. Because the compositions disclosed herein are resistant to forming fine powders, they deter abuse by inhalation.

Additionally, the compositions, whether whole or crushed/pulverized, form viscous colloidal dispersions when contacted with a small volume of a suitable solvent. The volume may be about 3 mL, 5 mL, or 10 mL. Suitable solvents include water, alcohol solutions, acid mixtures, and the like. The viscosity of the dispersion prevents the material from being drawn through an injection syringe needle. Additionally, the dispersion comprises clay mineral particles that not only serve as visual deterrents, but also are difficult to remove by sedimentation or filtration and are large enough to restrict or block flow though injection needles. Consequently, the compositions disclosed herein are resistant to abuse by extraction, filtering, and/or injection.

### (II) Processes for Preparing Solid Dosage Pharmaceutical Compositions

Another aspect of the disclosure encompasses processes for preparing solid dosage forms of the pharmaceutical compositions disclosed herein. The processes comprise: (a) forming a mixture comprising at least one low molecular weight water-soluble polymer, at least one polyglycol, at least one polysaccharide, at least one clay mineral, and, optionally, an effervescent system; (b) forming a solid dosage unit from the mixture; and (c) heating the solid dosage unit to form the solid dosage form.

### (a) Forming a mixture

The first step of the process comprises forming a mixture comprising the components of the pharmaceutical composition, which are detailed above in section (I)(a). The mixture comprises at least one low molecular weight water-soluble polymer, at least one polyglycol, at least one polysaccharide, at least one clay mineral, a lubricant, and, optionally, an effervescent system. In general, the mixture further comprises at one API or a pharmaceutically acceptable salt thereof. The mixture components may be combined in any order or may be premixed in various combinations before being combined together. For example, the polyglycol and the clay mineral may be blended together before being combined with the rest of the components. Similarly, the API may be combined with some of the components before being combined with the rest of the components. Thus, a variety of ordered mixing schemes are possible.

In embodiments in which an effervescent formulation is prepared, the mixture may be prepared by first mixing (e.g., hot-melt granulating) the acid component of the effervescent system with the polyglycol to form a plurality of polyglycol-coated acid component particles. Next, the plurality of polyglycol-coated acid particles may be mixed with the clay mineral to form a plurality of clay mineral/polyglycol-coated particles. Lastly, the plurality of clay mineral/polyglycol-coated particles may be mixed with the rest of the components. The clay mineral/polyglycol coating may protect the acid component of the effervescent system from moisture and/or premature contact with the base component of the effervescent system (see Example 3 below).

The mixture comprising the components of the composition may be formed by mixing, roller mixing, drum mixing, shear mixing, dry blending, chopping, milling, granulating, dry granulating (e.g., slugging or roller compacting), wet granulating (e.g., fluid bed granulating, high shear granulating), and other mixing techniques known in the art.

### (b) Forming a solid dosage unit

The process further comprises forming the mixture from step (a) into a solid dosage unit. Suitable solid dosage units are described above in section (1)(b). Means of forming solid dosage units are well known in the art. In exemplary embodiments, the solid dosage unit may be a tablet. The tablet may be a compression tablet, a molded tablet, a compacted tablet, or a pressed tablet. In an exemplary embodiment, the tablet may be formed by direct compression. The shape of the tablet may vary. Non-limiting tablet shapes include round, oval, rectangular, and triangular. The size and mass of the tablet may vary. In various embodiments, the mass of the tablet may be range from about 100 mg to about 1000 mg.

### (c) Heating the solid dosage unit

The final step of the process comprises heating the solid dosage unit to yield the solid dosage form. This heating step dries and cures the solid dosage unit, wherein the cured solid dosage form may have improved properties or characteristics relative to an uncured solid dosage unit. For example, the heating step removes water from the solid dosage unit, therefore protecting the effervescent system in the solid dosage form from premature effervescence. The heating step may plasticize the low molecular weight water-soluble polymer(s) and/or melt the polyglycol, thereby forming a porous matrix solid dosage form that has increased resistant to crushing/pulverization as well as more rapid release of the API.

In general, the solid dosage unit is heated at a temperature of less than about 90°C. In various embodiments, the solid dosage unit may be heated at a temperature from about 30°C to about 35°C, from about 35°C to about 40°C, from about 40°C to about 45°C, from about 45°C to about 50°C, from about 50°C to about 55°C, from about 55°C to about 60°C, from about 60°C to about 65°C, from about 65°C to about 70°C, from about 70°C to about 75°C, from about 75°C to about 80°C, from about 80°C to about 85°C, or from about 85°C to about 90°C. In exemplary embodiments, the heating temperature may range from about 50°C to about 80°C.

The duration of the heating step can and will vary depending upon the components of the composition. The duration of the heating step may range from about 10 minutes to about 10 hours. In general, the higher the temperature, the shorter the duration of time. In an exemplary embodiment, the solid dosage unit may be heated to a temperature from about 60°C to about 80°C for a period of time ranging from about 1 hour to about 2 hours.

### EXAMPLES

The following examples are included to illustrate, but not to limit the claimed pharmaceutical compositions and processes for making.

### Example 1: Test Formulations

Two non-effervescent formulations (1, 2) and two effervescent (3, 4) formulations were prepared and tested for immediate release dissolution behavior and abuse deterrent (or tamper resistant) properties.

The components listed for each formulation were dry blended, directly compressed into tablets using a single station hand press (Natoli Engineering, St. Charles, MO) and a compression force of 425-475 units, and cured at 60-80°C for 1-2 hr.

The *in vitro* dissolution of oxycodone from the tablets was measured in 500 mL phosphate buffer or water using an USP Apparatus 2 (paddles) with a paddle speed of 50 rpm and a temperature of 37°C. The tablets were placed in sinkers to prevent flotation. Samples were removed at 15, 30, and 45 min and analyzed by HPLC for oxycodone hydrochloride.

Tamper resistance was tested by subjecting the tablets to grinding and milling tests. For the grinding test, a tablet was placed between two aluminum sample pans and hit ten times to crush the tablet into a thin pancake. The pancaked tablet was then manually crumbled (it was observed whether the material was waxy and or sticky and how easy it was to break the tablet into smaller fragments). The crumbles were then placed in a porcelain mortar and triturated (the material was monitored for pulverization, pilling, flaking, and/or sticking). Milling was only performed on tablets that show positive results for the grinding test. For the milling test, a tablet was placed in a hardness tester (Dr. Schleuniger Pharmatron, Manchester, NH; Model 6D) and flattened to a pancake. The flattened tablet was collected and placed in an analytical mill (IKA Laboratories; 10A mill) and milled for 1 minute intervals for 1, 3, 6, 9 min. The particle size was determined using standard means.

Table 1 presents the composition of non-effervescent formulation 1. Table 2 present the *in vitro* dissolution data. Tamper resistance tests revealed that the formulation 1 tablets could not be pulverized, but formed waxy flaky materials when subjected to trituration.

| **Table 1.** Composition of Non-effervescent Formulation 1. | |
|---|---|
| **Ingredient** | **% (w/w) of Composition** |
| Oxycodone | 21 |
| PEO 100,000 g/mol | 20 |
| Pluronic F68 | 35 |
| Xanthan Gum | 3 |
| Sodium Bentonite | 10 |
| F-Melt | 10 |
| Magnesium Stearate | 1 |

| **Table 2.** Dissolution of Formulation 1. | | |
|---|---|---|
| Time | Water (%) | Phosphate Buffer (%) |
| 15 min | 53.5 | 49.9 |
| 30 min | 79.4 | 82.5 |
| 45 min | 87.5 | 97.8 |

Table 3 presents the composition of non-effervescent formulation 2. Table 4 shows that this formulation clearly met the standard for immediate release (i.e., at least 70% released within 30 min or 45 min). Tamper resistance tests revealed that the tablets could not be pulverized, but formed waxy flaky materials when subjected to crushing.

| **Table 3.** Composition of Non-effervescent Formulation 2. | |
|---|---|
| **Ingredient** | **% (w/w) of Composition** |
| Oxycodone | 20 |
| PEO 100,000 g/mol | 26 |
| Pluronic F68 | 9.5 |
| Pluronic F127 | 9.5 |
| Xanthan Gum | 3 |
| Sodium Bentonite | 16 |
| Micronized Talc | 5 |
| Crospovidone XL-10 | 10 |
| Magnesium Stearate | 1 |

| **Table 4.** Dissolution of Formulation 2. | | |
|---|---|---|
| Time | Water (%) | Phosphate Buffer (%) |
| 15 min | - | 66.7 |
| 30 min | - | 88.2 |
| 45 min | - | 96.1 |

Table 5 presents the composition of effervescent formulation 3, and Table 6 shows that this formulation had immediate release. The tablets were hard and plastic. When milled for 9 min, 66% of the tablet retained a particle size of greater than 500 microns.

| **Table 5.** Composition of Effervescent Formulation 3. | |
|---|---|
| **Ingredient** | **% (w/w) of Composition** |
| Oxycodone | 21 |
| PEO 100,000 g/mol | 20 |
| Pluronic F68 | 19.25 |
| Xanthan Gum | 3 |
| Sodium Bentonite | 10 |
| Effersoda | 13.75 |
| Citric Acid | 4 |
| Tartaric Acid | 8 |
| Magnesium Stearate | 1 |

| **Table 6.** Dissolution of Formulation 3. | | |
|---|---|---|
| Time | Water (%) | Phosphate Buffer (%) |
| 15 min | 68.0 | 71.7 |
| 30 min | 87.4 | 94.2 |
| 45 min | 88.8 | 95.8 |

Table 7 presents the composition of effervescent formulation 4, and Table 8 presents the *in vitro* release data. The tablets were hard and plastic and could not be pulverized, but formed waxy flaky materials when triturated. When milled for 9 min, 66% of the tablet retained a particle size of greater than 500 microns.

| **Table 7.** Composition of Effervescent Formulation 4. | |
|---|---|
| **Ingredient** | **% (w/w) of Composition** |
| Oxycodone | 21 |
| PEO 100,000 g/mol | 30 |
| Pluronic F68 | 10 |
| Xanthan Gum | 3 |
| Sodium Bentonite | 15 |
| Sodium Bicarbonate | 10.58 |
| Tartaric Acid | 9.42 |
| Magnesium Stearate | 1 |

| **Table 8.** Dissolution of Formulation 4. | | |
|---|---|---|
| Time | Water (%) | Phosphate Buffer (%) |
| 15 min | 73.1 | 86.0 |
| 30 min | 83.0 | 97.5 |
| 45 min | 83.3 | 98.2 |

### Example 2: Effect of Individual Components on Tablet Performance

The effective concentration range of each individual component was analyzed in non-effervescent and effervescent formulations. The formulations were prepared and formed into tablets essentially as detailed above in Example 1. The disintegration was tested using the standard paddle dissolution apparatus as detailed in Example 1, except disintegration was monitored at 30 min. At 30 min, any remaining tablet was removed from the sinker, wiped off and weighed on a standard balance to determine how much of the tablet was remaining. If the tablet had completely disintegrated before the 30 min mark, the time to complete disintegration was recorded instead. The tamper resistance was monitored using the grinding and milling tests detailed above in Example 1. The following scale was used to rank tamper resistance (0 = little or no resistance, 6 = excellent tamper resistance):
0 - Tablets showed processing issues such as picking and capping.
1 - Tablet is crumbly or brittle and can be completely or partially pulverized.
2 - Tablet is crumbly but forms flakes when attempting to pulverize.
3 - Tablet is crumbly but forms waxy and/or sticky flakes when attempting to pulverize.
4 - Tablet is not crumbly but breaks down to large fakes when attempting to pulverize.
5 - Tablet is plastic and does not break down by trituration.
6 - Tablet is plastic and does not break down by trituration and does not pulverize through milling.

The results are shown below in Tables 9-16. The results are bracketed according to percent ranges. Abbreviations used in the tables are: T&B (talc and bentonite combined percentage), XG (xanthan gum), CA (citric acid), Man (mannitol), SB (co-processed sorbitol/glycerol), SA (sodium alginate), rem. (remaining), TR (tamper resistance).

*Polyox (PEO) 100.000 g*/*mol.* Formulations were prepared that contained from 0-66% (w/w) of Polyox 100,000 g/mol. Tables 9 and 10 show the results for non-effervescent and effervescent formulations, respectively. These studies revealed that Polyox 100,000 g/mol provided hardness and plasticity to the tablets.

| **Table 9.** Effect of Various Concentrations of Polyox on Non-Effervescent Formulations. | | | | | |
|---|---|---|---|---|---|
| Polyox Range | Tamper Resistance | | Disintegration | | |
| | TR rating | Exceptions | Approximate Amount Remaining | Exceptions | % dissolution in water at 30 min |
| 50% | 5 | > 3% XG and/or > 5% talc and/or > 5% sodium bentonite caused inadequate TR performance | > 50% | 30% rem. if 50% Polyox with 5% talc and 10% bentonite (TR properties insufficient) | Not tested |
| ≥ 40% | 3 | All samples tested in this range contained high levels of T&B. 3 samples w/out T&B had better TR properties | Mostly > 50% | No CA, no Man, ≥ 7.5% bentonite - < 16 % rem. (TR properties insufficient) | 50 - 65% |
| ≥ 35% | 3 | All samples tested in this range contained high levels of T&B. Either no talc or no bentonite or no CA, no Man resulted in better TR performance | > 30% | Only F68 used. No F127 | 50% |
| ≥ 30% | 4 | Incorporation of more than 6% XG, and/or > 1% CA, and/or contained Pullulan, and/or contained SB, and/or contained man, and/or contained SA causes poorer TR performance | Mostly > 25% | 42% F68, no F127, 0% T&B (good TR properties) | 75 - 85% |
| | | | | 38% F68, no F127, 5% bentonite. (TR properties insufficient) | |
| | | | | High T&B (5/13.5%) (TR properties insufficient) | |
| ≥ 20% | 2 | No F-Melt in combination with high T&B and/or w/ XG 200 mesh, or Pullulan or CA, and/or T&B ≥ 20% results in worse TR properties | Mostly 20% < rem. < 30% | 35% F68, no F127, no talc, 5% F-Melt (good TR properties) | 75 - 80% |
| | | | | 32% F68, no F127, no talc, 19% bentonite (good TR properties) | |
| | | | | 33% F68, no F127, no talc, 10% F-Melt (good TR properties) | |
| < 20% | 0, 1, or 2 | No Exceptions | Not tested | None | Not tested |
| 0% | 1 | No Exceptions | > 60% | None | Not tested |

| **Table 10.** Effect of Various Concentrations of Polyox on Effervescent Formulations. | | | | | |
|---|---|---|---|---|---|
| Polyox Range | Tamper Resistance | | Disintegration | | |
| | TR rating | Exceptions / Explanations | Approximate Amount Remaining | Exceptions | % dissolution in water at 30 min |
| ≥ 40% | 5 | No Exceptions | Not Tested | N/A | 50 - 65% |
| ≥ 30% | 3, 4 | 3 for samples not containing CA and/or with < 32% Polyox | Fast disintegration if at least 20% of effervescent materials used | none | 40 - 50% for low effervescent levels, >80% for high effervescent levels |
| ≥ 20% | 2, 4 | 2 for samples not containing CA | Fast disintegration if at least 20% of effervescent materials used | Low effervescent levels not tested | >87% |

*Pluronics.* Formulations were prepared in which the total concentration of Pluronic ranged from 0 - 46% (w/w). Tables 11 and 12 present the tamper resistance and disintegration data for non-effervescent and effervescent formulations, respectively. Pluronics provided waxy properties to the tablets and aided in the formation of the non-filterable particle dispersion. It was found that disintegration and dissolution were highly dependent on the choice of Pluronic (i.e., the tested Pluronics had the following order, from the slowest to the fastest: F127 < F108 < F87 ≤ F68). It was found that Pluronic levels below 10% negatively affected the strength of particle suspension.

| **Table 11.** Effect of Various Concentrations of Pluronics on Non-Effervescent Formulations. | | | | | |
|---|---|---|---|---|---|
| Pluronic Range | Tamper Resistance | | Disintegration | | |
| | TR rating | Explanations | Approxima te Amount Remaining after 30 min | Exceptions / Explanations | % dissolution in water at 30 min |
| ≥ 34% F68 only | 3 - 4 | High Pluronic levels improve waxy properties of the tablets. High Polyox levels are however needed to provide | 10% | None | 80 - 85% |
| | | sufficient plasticity | | | |
| | 1 - 2 | Samples containing CA, Pullulan, 200 Mesh XG or > 3% XG 60 Mesh or ≥ 20% bentonite are more brittle | 13% | Samples containing > 25% Bentonite and > 4% XG disintegrated more slowly | 45 - 75% |
| ≥ 30% F68 only | 4 | None | 10 - 33% | None | 75 - 80% |
| | 0, 2 | Samples containing CA, Pullulan, 200 mesh XG or > 3% XG 60 mesh or ≥ 20% bentonite are more brittle | 15 - 20% | None | 65 - 80% |
| ≥ 20% | 3 | None | 33 - 50% or 20% | 33 - 50% for samples with 1:1 F68:F127, 20% for samples with only F68 | 50 - 60% (only F68 samples) |
| | 0, 2 | F68 only. Samples with 0 rating contained 19% Bentonite and CA | Not tested | N/A | Not tested (disintegra tion too slow) |
| ≥ 10% 1:1 F127:F68 | 4 - 5 | None | 80% | Samples containing no T&B | Not tested (disintegra tion too slow) |
| | | | 20 - 60% | Samples containing talc and/or bentonite | |
| | 0, 1 | 0 ratting-10-15% talc 1 rating - T&B > 15% or contained CA | 5 - 50% | 50% for samples containing Na salts | 50 - 90% |
| < 10% | 5 | All formulations contained > 50% Polyox and 3% CA and ≤ 5% bentonite | > 60% | None | Not tested |
| 0% | 4 | Formulations had close to 30% bentonite | Not extensively tested | None | 50% |

| **Table 12.** Effect of Various Concentrations of Pluronics on Effervescent Formulations. | | | | | |
|---|---|---|---|---|---|
| Pluronics Range | Tamper Resistance | | Disintegration | | |
| | TR rating | Explanations | Approxima te Amount Remaining after 30 min | Exceptions / Explanations | % Dissolution in water at 30 min |
| ≥ 20% | 5 | Contained 20 - 40% Polyox | 15 - 20% | Contained > 20% effervescent materials | Not tested |
| | | | 33% | Contained < 10% effervescent materials | 50% |
| ≥ 15% | 5 | Contained 20 - 40% Polyox | 0% | Contained > 25% effervescent materials | > 85% |
| | | | 30% | Contained < 10% effervescent materials | 50% |
| ≥ 10% | 4 - 6 | Contained 25 - 50% Polyox. Sample with a 6 rating contained 10% F68, 35% Polyox, 20% effervescent materials | 0% | Contained > 25% effervescent materials | > 80% |
| | | | 15 - 30% | Contained ≤ 20% effervescent materials or contained > 25% effervescent materials but uses F127 instead of F68 | 50 - 75% |
| < 10% | 5 - 6 | Contained 30 - 36% Polyox. | 0 - 15% | None | 80 - 85% |

*Xanthan Gum.* Concentration test range was from 0-12% (w/w) in the formulations. Table 14 presents the results for the non-effervescent formulations. It was found that higher xanthan gum concentrations incorporated in the formulations, resulted in stronger suspensions with greater abuse deterrent properties. A minimum of 3% (w/w) was needed in the formulation. However, the level of xanthan gum inversely correlated with disintegration and dissolution rate at levels > 3%.

| **Table 13.** Effect of Various Concentrations of Xanthan Gum on Non-Effervescent Formulations. | | | | | |
|---|---|---|---|---|---|
| XG Range | Tamper Resistance | | Disintegration | | |
| | TR rating | Explanations | Approximate Amount Remaining after 30 min | Exceptions / Explanations | % dissoluti on in water at 30 min |
| > 6% | 1 - 2 | XG at high levels increases tablet brittleness | 50 - 70% | none | Not tested |
| 6% | 5 | Contained > 30% | 80% | 1 sample containing | Not |
| | | Polyox and 5% CA and no bentonite and talc | | 32% F68 and 10% bentonite achieved 20% rem. | tested |
| | 1 | Contained 10-15% talc or bentonite | 30-50% | Samples containing 10% bentonite, 30% F68, no talc | |
| >3% | 3 | Generally contained > 30% F68 and Polyox and not more than 10% bentonite | 15 - 30% | none | 75 - 85% |
| | 2 | Contain 30 - 45% F68 and 19 - 20% bentonite | 30 - 60% | none | 50% |
| 0-3% | varies | TR performance independent of XG levels ≤ 3% | varies | Disintegration and dissolution are governed by other components at XG levels ≤ 3% | 40-92% |

For effervescent formulations, xanthan gum levels were held constant at 3% (w/w) with two exceptions of 6%. It was found that 6% could be used, since high levels of Polyox allowed for incorporation of a larger quantity of materials such as xanthan gum, to offset the brittleness of the xanthan gum. These formulations however, showed insufficient dissolution, even with 20% effervescent materials (e.g., 73% in 30 min).

*Micronized Talc.* Concentration test range was from 0 -18% (w/w) in the formulations. Table 14 presents the tamper resistance and disintegration data for non-effervescent formulations. Talc worked as a glidant and reduced processing issues due to the Pluronics. Talc also improved tablet disintegration time but at high levels negatively impacted TR properties.

| **Table 14.** Effect of Various Concentrations of Micronized Talc on Non-Effervescent Formulations. | | | | | |
|---|---|---|---|---|---|
| Talc Range | Tamper Resistance | | Disintegration | | |
| | TR rating | Explanations | Approximate Amount Remaining after 30 min | Exceptions / Explanations | % dissoluti on in water at 30 min |
| ≥ 15% | 2 | High levels of Talc impart brittleness | 15 - 60% | 15% achieved through use of 30% F68 and 23% Polyox with 18% talc | Not tested |
| ≥ 10% | 3 | Contained at least 29% | 30 - 50% | None | 75% |
| | | Polyox and ≤ 20% of 1:1 F127:F68 | | | |
| | 2 | Either contained CA and Man or had < 30% Polyox and > 15% of 1:1 F127:F68 | 30 - 50% | Samples also containing 5-15% bentonite and < 28% Polyox had 0 - 20% rem. at 30 min | Not tested |
| ≥ 5% | 4 | Contained > 50% Polyox and 3% CA and 5-10% Pluronics or 23 - 33% Polyox and 23% Pluronics and no CA | 50 - 75% | Samples containing 23 - 33% Polyox had 20 - 30% rem. | Not tested |
| | 2 | Samples also contained 5-18% bentonite or > 3% XG | 30 - 60% | Contained 3-5% CA and 5-10% Man | 40-75% |
| | | | 0 - 20% | Did not contain CA or Man | 65 - 92% |
| 0-5% | varies | TR not markedly affected by talc itself in this range | 0 - 80% | Disintegration not significantly driven by talc in this range | 50-85% |

Only a 5% level of talc was investigated in effervescent tablets. In these formulations, Polyox levels were at 25 - 35% and effervescent materials were present at > 40%. TR performance for these tablets was ranked at 4 - 5 with complete disintegration being achieved at 30 min.

*Sodium Bentonite.* The concentration range tested was 0-32% (w/w). Tables 15 and 16 present the tamper resistance and disintegration data for non-effervescent and effervescent formulations, respectively. Sodium bentonite worked somewhat as a glidant, improving powder flow in bulk blends. Due to its nature of swelling to multiple times its dry volume in water, it also improved tablet disintegration time. At high levels however it negatively impacted TR properties.

| **Table 15.** Effect of Various Concentrations of Sodium Bentonite on Non-Effervescent Formulations. | | | | | |
|---|---|---|---|---|---|
| Bentonite Range | Tamper Resistance | | Disintegration | | |
| | TR rating | Explanations | Approximat e Amount Remaining after 30 min | Exceptions / Explanations | % dissolution in water at 30 min |
| ≥ 20% | 0,2 | High bentonite levels impart brittleness to tablets | Not tested | Poor intactness of tablets rendered disintegration and dissolution testing invaluable | Not tested |
| ≥ 15% | 2 | All samples contained 15% | 20 - 50% | None | 75% |
| | | bentonite and 18 - 23% Pluronics or 19% bentonite and 32% Pluronics | | | |
| | 1, 3 | 1 - Samples containing < 30% Pluronics 3 - Samples containing > 30% Pluronics | 0-33% | Samples containing > 3% XG were slower (66%) | 45 - 80% |
| ≥ 10% | 3 - 4 | Less brittle, no crumbling | 6 - 33% | Disintegration and dissolution largely governed by other excipients | 50 - 80% |
| ≥ 5% | 3 - 5 | Less brittle, no crumbling except for samples with talc > 5% | 20 - 60% | Tablets with talc> 5% performed better (15% rem) | 50 - 75% (not extensivel y studied) |
| 0% | 4 - 5 | Generally good TR properties. Contained 45 - 50% Polyox, except one sample with 32% Polyox and 42% Pluronics | 30 - 80% | High Polyox causes slow disintegration | 85% (exception described under TR Explanatio ns) |

| **Table 16.** Effect of Various Concentrations of Sodium Bentonite on Effervescent Formulations. | | | | | |
|---|---|---|---|---|---|
| Bentonite Range | Tamper Resistance | | Disintegration | | |
| | TR rating | Explanations | Approximate Amount Remaining after 30 min | Exceptions / Explanations | % dissolution in water at 30min |
| >10% | 5 | Samples contained ≥ 30% Polyox and ≤ 10% F68 | 0 - 80% | Disintegration time was highly dependent on effervescent material levels | 83% for samples 20% effervescent materials |
| 10% | 5 | Samples with > 30% Polyox had plastic properties. Samples with < 30% Polyox contained ≥ 4% CA | 0 - 20% | Samples with only CA and no TA or with Polyox > 34% showed slower disintegration | 87% for a Sample with 2:1 TA:CA and 20% Polyox and 20% F68 |
| | 4 | Contained only TA (no CA) | 0 - 33% | Disintegration and dissolution are mainly governed by effervescent material levels | 50 - 85% |
| < 10% | 5 | All samples contained 25 - 50% Polyox and ≤ 10% Pluronics | 0 - 40% | Disintegration and dissolution are mainly governed by | 65 - 85% |
| | | | | effervescent material levels | |

### Example 3: Stabilizing the Effenvescent Components

The effervescent formulations are susceptible to premature effervescence under conditions of high humidity. Such formulations may have a reduced shelf-life and decreased stability. The following example details a process for coating the acid component of an effervescent system to reduce moisture sensitivity and lower the likelihood of premature effervescence.

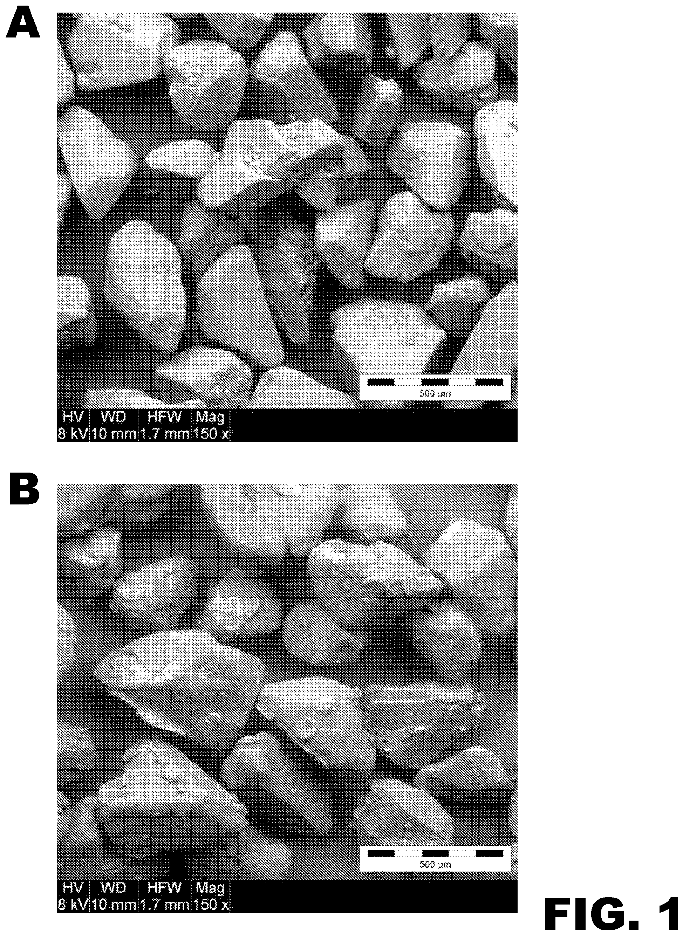

L-(+)-tartaric acid was hot-melt granulated with Kolliphor P 407 (Pluronic F127). The materials were blended in a water-jacketed granulator until the product temperature reached 60°C. The material was then removed from the granulation bowl and allowed to cool to room temperature, at which point it was sieved through a 20 mesh screen to break down any agglomerates. **FIG. 1A** presents a SEM image of L-(+)-tartaric acid particles and **FIG. 1B** presents a similar image of tartaric acid particles that are evenly coated with Pluronic F127. The coating appears have some fractures, which are likely due to the breaking of agglomerates during the sieving process.

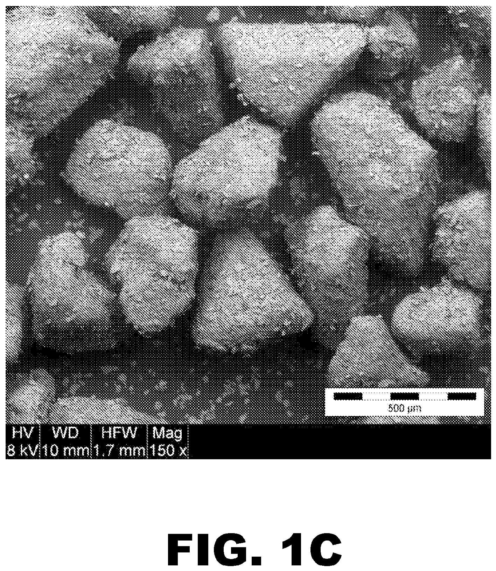

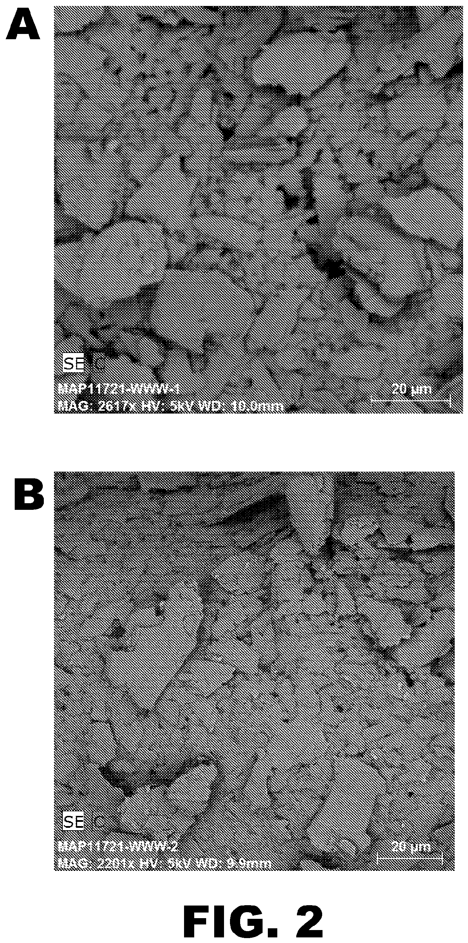

The Pluronic-coated tartaric acid particles were then blended with talc, which sticks to the surface of the particles. **FIG. 1C** present a SEM image of the talc-coated particles. Elemental mapping of the coated particles revealed that the majority of the particles' surfaces were covered with talc, with limited Pluronic F127-coated surfaces visible (see **FIG. 2**).

The Pluronic F127-talc coating formed a moisture barrier over the tartaric acid particles, preventing interaction of tartaric acid with water and sodium bicarbonate. Dynamic vapor sorption studies (DVS) revealed that tablets comprising the coated tartaric acid particles had less moisture sensitivity that tablets comprising regular tartaric acid. The DVS data are presented in Table 17. Weight gain indicates moisture sorption by the tablet, and weight loss indicates the onset of the stability-compromising effervescent reaction. These results show that the use of the coated tartaric acid allowed for more moisture sorption to occur without effervescence being initiated immediately. Even when weight loss was initiated in the formulation containing the tartaric acid coated with Pluronic, the weight loss was slower than in the formulation with regular tartaric acid. These data indicate that formulations comprising coated tartaric acid particles had slower effervescent reactions during tablet storage and the coating provided greater moisture protection.

**Table 17. Dynamic Vapor Sorption Results.**

| **Condition** | **Regular tartaric acid and sodium bicarbonate** | **Coated tartaric acid and sodium bicarbonate** |
|---|---|---|
| **40°C, 50% RH** | 1.3% total weight gain over 50 hrs | 1.2% weight gain over 36 hrs |
| **40°C, 60% RH** | 2.3% total weight gain followed by 3.5% total weight loss over 40 hrs | 2.4% weight gain over 11.4 hrs |
| **40°C, 67% RH** | 0.8% total weight gain followed by 3.4% weight loss over 17 hrs | 3.5% weight gain followed by 2% weight loss over 67 hrs |

## Claims

1. A pharmaceutical composition comprising at least one active pharmaceutical ingredient (API) having a potential for abuse or a pharmaceutically acceptable salt thereof, at least one low molecular weight water-soluble polymer having a molecular weight of no more than 300,000 daltons, at least one polyglycol, at least one polysaccharide, at least one clay mineral, and, optionally, an effervescent system, wherein the pharmaceutical composition is an oral solid dosage form.

2. The pharmaceutical composition of claim 1, wherein the low molecular weight water-soluble polymer is chosen from a polyalkylene oxide, a cellulose ether, a polyolefinic alcohol, a polyvinyl lactam, a polycarboxylic acid, and combinations thereof; and the low molecular weight water-soluble polymer is present in an amount from 5% to 60% by weight of the pharmaceutical composition.

3. The pharmaceutical composition of either claim 1 or claim 2, wherein the polyglycol is chosen from a copolymer of ethylene glycol and propylene glycol, a polyethylene glycol, a polypropylene glycol, and combinations thereof; and the polyglycol is present in an amount from 5% to 50% by weight of the pharmaceutical composition.

4. The pharmaceutical composition of any of claims 1 to 3, wherein the polysaccharide is a gum chosen from xanthan gum, acacia gum, diutan gum, gellan gum, guar gum, fenugreek gum, locust bean gum, pullulan, welan gum, and combinations thereof; and the polysaccharide is present in an amount from 1% to 10% by weight of the pharmaceutical composition.

5. The pharmaceutical composition of any of claims 1 to 4, wherein the clay mineral is chosen from a bentonite, talc, and combinations thereof; and the clay mineral is present in an amount from 1% to 30% by weight of the pharmaceutical composition.

6. The pharmaceutical composition of any of claims 1 to 5, wherein the effervescent system comprises a) an acid component chosen from an organic acid, an inorganic acid, and combinations thereof and b) a base component chosen from an alkali metal bicarbonate, an alkaline earth metal bicarbonate, an alkali metal carbonate, an organic carbonate, and combinations thereof; and when the effervescent system is present in the pharmaceutical composition, it is present in an amount from 5% to 70% by weight of the pharmaceutical composition.

7. The pharmaceutical composition of any of claims 1 to 6, wherein the API is an opioid or a combination of an opioid and a non-opioid analgesic; and the opioid is chosen from oxycodone, oxymorphone, hydrocodone, hydromorphone, codeine, and morphine.

8. The pharmaceutical composition of any of claims 1 to 7, further comprising a lubricant and, optionally, a superdisintegrant.

9. The pharmaceutical composition of any of claims 1 to 8, wherein the pharmaceutical composition releases at least 70% of the API within 45 minutes when measured using an USP-approved *in vitro* release procedure.

10. The pharmaceutical composition of any of claims 1 to 9, wherein when the pharmaceutical composition is milled for 9 minutes to form particles, more than 30% of the particles have an average diameter of greater than 250 microns.

11. The pharmaceutical composition of any of claims 1 to 10, wherein a viscous gel and/or colloidal dispersion is formed when the pharmaceutical composition is contacted with a small volume of an aqueous solvent.

12. The pharmaceutical composition of any of claims 1 to 11, wherein the low molecular weight water-soluble polymer is a polyethylene oxide having a molecular weight of 100,000 daltons and is present in an amount from 20% to 50% by weight of the pharmaceutical composition; the polyglycol is a copolymer of ethylene glycol and propylene glycol and is present in an amount from 10% to 45% by weight of the pharmaceutical composition; the polysaccharide is xanthan gum and is present in an amount from 2% to 6% by weight of the pharmaceutical composition; the clay mineral is sodium bentonite or a combination of sodium bentonite and talc and is present in an amount from 5% to 25% by weight of the pharmaceutical composition; and the API is an opioid chosen from oxycodone, oxymorphone, hydrocodone, hydromorphone, codeine, and morphine.

13. The pharmaceutical composition of claim 12, further comprising the optional effervescent system comprising a) an acid component chosen from an organic acid, an inorganic acid, and combinations thereof and b) a base component chosen from an alkali metal bicarbonate, an alkaline earth metal bicarbonate, an alkali metal carbonate, an organic carbonate, and combinations thereof; and the effervescent system is present in an amount from 15% to 50% by weight of the pharmaceutical composition.

14. A process for preparing an oral solid dosage form of the pharmaceutical composition of any of claims 1 to 13, wherein the process comprises:
a) forming a mixture comprising the at least one active pharmaceutical ingredient (API) having a potential for abuse or pharmaceutically acceptable salt thereof, the at least one low molecular weight water-soluble polymer having a molecular weight of no more than 300,000 daltons, the at least one polyglycol, the at least one polysaccharide, the at least one clay mineral, and the optional effervescent system;
b) forming the mixture into a solid dosage unit; and
c) heating the solid dosage unit to yield the oral solid dosage form.

## Patentansprüche

1. Pharmazeutische Zusammensetzung, umfassend wenigstens einen pharmazeutischen Wirkstoff (API) mit Missbrauchspotenzial oder ein pharmazeutisch verträgliches Salz davon, wenigstens ein niedermolekulares wasserlösliches Polymer mit einem Molekulargewicht von nicht mehr als 300.000 Dalton, wenigstens ein Polyglycol, wenigstens ein Polysaccharid, wenigstens ein Tonmineral und, optional, ein Brausesystem, wobei die pharmazeutische Zusammensetzung eine feste orale Dosierungsform ist.

2. Pharmazeutische Zusammensetzung nach Anspruch 1, wobei das niedermolekulare wasserlösliche Polymer ausgewählt ist aus einem Polyalkylenoxid, einem Celluloseether, einem polyolefinischen Alkohol, einem Polyvinyllactam, einer Polycarbonsäure und Kombinationen davon; und das niedermolekulare wasserlösliche Polymer in einer Menge von 5 bis 60 Gew.-% der pharmazeutischen Zusammensetzung vorliegt.

3. Pharmazeutische Zusammensetzung nach entweder Anspruch 1 oder Anspruch 2, wobei das Polyglycol ausgewählt ist aus einem Copolymer von Ethylenglycol und Propylenglycol, einem Polyethylenglycol, einem Polypropylenglycol und Kombinationen davon; und das Polyglycol in einer Menge von 5 bis 50 Gew.-% der pharmazeutischen Zusammensetzung vorliegt.

4. Pharmazeutische Zusammensetzung nach einem der Ansprüche 1 bis 3, wobei das Polysaccharid ein Gummi ist, das ausgewählt ist aus Xanthangummi, Akaziengummi, Diutangummi, Gellangummi, Guargummi, Fenugreek-Gummi, Johannisbrotgummi, Pullulan, Welangummi und Kombinationen davon; und das Polysaccharid in einer Menge von 1 bis 10 Gew.-% der pharmazeutischen Zusammensetzung vorliegt.

5. Pharmazeutische Zusammensetzung nach einem der Ansprüche 1 bis 4, wobei das Tonmineral ausgewählt ist aus einem Bentonit, Talk und Kombinationen davon; und das Tonmineral in einer Menge von 1 bis 30 Gew.-% der pharmazeutischen Zusammensetzung vorliegt.

6. Pharmazeutische Zusammensetzung nach einem der Ansprüche 1 bis 5, wobei das Brausesystem umfasst: a) eine Säurekomponente, die ausgewählt ist aus einer organischen Säure, einer anorganischen Säure und Kombinationen davon, und b) eine Basenkomponente, die ausgewählt ist aus einem Alkalimetallbicarbonat, einem Erdalkalimetallbicarbonat, einem Alkalimetallcarbonat, einem organischen Carbonat und Kombinationen davon; und das Brausesystem, wenn es in der pharmazeutischen Zusammensetzung vorliegt, in einer Menge von 5 bis 70 Gew.-% der pharmazeutischen Zusammensetzung vorliegt.

7. Pharmazeutische Zusammensetzung nach einem der Ansprüche 1 bis 6, wobei der API ein Opioid oder eine Kombination aus einem Opioid und einem Nichtopioid-Analgetikum ist; und das Opioid ausgewählt ist aus Oxycodon, Oxymorphon, Hydrocodon, Hydromorphon, Codein und Morphin.

8. Pharmazeutische Zusammensetzung nach einem der Ansprüche 1 bis 7, weiterhin umfassend ein Gleitmittel und, optional, ein Superzerfallsmittel.

9. Pharmazeutische Zusammensetzung nach einem der Ansprüche 1 bis 8, wobei die pharmazeutische Zusammensetzung wenigstens 70 % des API innerhalb von 45 Minuten freisetzt, gemessen nach einer gemäß USP zugelassenen *In-vitro-*Freisetzungsmethode.

10. Pharmazeutische Zusammensetzung nach einem der Ansprüche 1 bis 9, wobei mehr als 30 % der Teilchen einen mittleren Durchmesser von mehr als 250 Mikron haben, wenn die pharmazeutische Zusammensetzung zur Bildung von Teilchen 9 Minuten lang gemahlen wird.

11. Pharmazeutische Zusammensetzung nach einem der Ansprüche 1 bis 10, wobei ein viskoses Gel und/oder eine kolloidale Dispersion gebildet werden, wenn die pharmazeutische Zusammensetzung mit einem kleinen Volumen eines wässrigen Lösemittels in Kontakt gebracht wird.

12. Pharmazeutische Zusammensetzung nach einem der Ansprüche 1 bis 11, wobei das niedermolekulare wasserlösliche Polymer ein Polyethylenoxid mit einem Molekulargewicht von 100.000 Dalton ist und in einer Menge von 20 bis 50 Gew.-% der pharmazeutischen Zusammensetzung vorliegt; das Polyglycol ein Copolymer von Ethylenglycol und Propylenglycol ist und in einer Menge von 10 bis 45 Gew.-% der pharmazeutischen Zusammensetzung vorliegt; das Polysaccharid Xanthangummi ist und in einer Menge von 2 bis 6 Gew.-% der pharmazeutischen Zusammensetzung vorliegt; das Tonmineral Natriumbentonit oder eine Kombination aus Natriumbentonit und Talk ist und in einer Menge von 5 bis 25 Gew.-% der pharmazeutischen Zusammensetzung vorliegt; und der API ein Opioid ist, das ausgewählt ist aus Oxycodon, Oxymorphon, Hydrocodon, Hydromorphon, Codein und Morphin.

13. Pharmazeutische Zusammensetzung nach Anspruch 12, weiterhin umfassend das optionale Brausesystem umfassend: a) eine Säurekomponente, die ausgewählt ist aus einer organischen Säure, einer anorganischen Säure und Kombinationen davon, und b) eine Basenkomponente, die ausgewählt ist aus einem Alkalimetallbicarbonat, einem Erdalkalimetallbicarbonat, einem Alkalimetallcarbonat, einem organischen Carbonat und Kombinationen davon; und das Brausesystem in einer Menge von 15 bis 50 Gew.-% der pharmazeutischen Zusammensetzung vorliegt.

14. Verfahren zur Herstellung einer festen oralen Dosierungsform der pharmazeutischen Zusammensetzung nach einem der Ansprüche 1 bis 13, wobei das Verfahren umfasst:
a) Bilden einer Mischung, die den wenigstens einen pharmazeutischen Wirkstoff (API)) mit Missbrauchspotenzial oder ein pharmazeutisch verträgliches Salz davon, das wenigstens eine niedermolekulare wasserlösliche Polymer mit einem Molekulargewicht von nicht mehr als 300.000 Dalton, das wenigstens eine Polyglycol, das wenigstens eine Polysaccharid, das wenigstens eine Tonmineral und das optionale Brausesystem umfasst;
b) Formen der Mischung zu einer festen Dosierungseinheit; und
c) Erwärmen der festen Dosierungseinheit zum Erhalt der festen oralen Dosierungsform.

## Revendications

1. Composition pharmaceutique comprenant au moins un ingrédient pharmaceutique actif (IPA) ayant un potentiel pour les abus ou un sel pharmaceutiquement acceptable de celui-ci, au moins un polymère soluble dans l'eau de faible masse moléculaire ayant une masse moléculaire de pas plus de 300000 daltons, au moins un polyglycol, au moins un polysaccharide, au moins un minéral de type argile et, éventuellement, un système effervescent, où la composition pharmaceutique est une forme galénique solide orale.

2. Composition pharmaceutique selon la revendication 1, où le polymère soluble dans l'eau de faible masse moléculaire est choisi parmi un poly(oxyde d'alkylène), un éther de cellulose, un poly(alcool oléfinique), un polyvinyllactame, un poly(acide carboxylique), et leurs combinaisons; et le polymère soluble dans l'eau de faible masse moléculaire est présent en une quantité de 5 % à 60 % en poids de la composition pharmaceutique.

3. Composition pharmaceutique selon la revendication 1 ou la revendication 2, où le polyglycol est choisi parmi un copolymère d'éthylèneglycol et de propylèneglycol, un polyéthylèneglycol, un polypropylèneglycol, et leurs combinaisons; et le polyglycol est présent en une quantité de 5 % à 50 % en poids de la composition pharmaceutique.

4. Composition pharmaceutique selon l'une quelconque des revendications 1 à 3, où le polysaccharide est une gomme choisie parmi la gomme de xanthane, la gomme arabique, la gomme de diutane, la gomme de gellane, la gomme de guar, la gomme de fenugrec, la gomme de caroube, le pullulane, la gomme de welane, et leurs combinaisons; et le polysaccharide est présent en une quantité de 1 % à 10 % en poids de la composition pharmaceutique.

5. Composition pharmaceutique selon l'une quelconque des revendications 1 à 4, où le minéral de type argile est choisi parmi une bentonite, le talc, et leurs combinaisons; et le minéral de type argile est présent en une quantité de 1 % à 30 % en poids de la composition pharmaceutique.

6. Composition pharmaceutique selon l'une quelconque des revendications 1 à 5, où le système effervescent comprend a) un composant acide choisi parmi un acide organique, un acide inorganique, et leurs combinaisons et b) un composant base choisi parmi un bicarbonate de métal alcalin, un bicarbonate de métal alcalino-terreux, un carbonate de métal alcalin, un carbonate organique, et leurs combinaisons; et quand le système effervescent est présent dans la composition pharmaceutique, il est présent en une quantité de 5 % à 70 % en poids de la composition pharmaceutique.

7. Composition pharmaceutique selon l'une quelconque des revendications 1 à 6, où l'IPA est un opioïde ou une combinaison d'un opioïde et d'un analgésique non opioïde; et l'opioïde est choisi parmi l'oxycodone, l'oxymorphone, l'hydrocodone, l'hydromorphone, la codéine et la morphine.

8. Composition pharmaceutique selon l'une quelconque des revendications 1 à 7, comprenant en outre un lubrifiant et, éventuellement, un superdésintégrant.

9. Composition pharmaceutique selon l'une quelconque des revendications 1 à 8, où la composition pharmaceutique libère au moins 70 % de l'IPA en une durée de jusqu'à 45 minutes quand elle est mesurée au moyen d'un processus de libération *in vitro* approuvé par l'USP.

10. Composition pharmaceutique selon l'une quelconque des revendications 1 à 9, où quand la composition pharmaceutique est broyée pendant 9 minutes pour former des particules, plus de 30 % des particules ont un diamètre moyen supérieur à 250 micromètres.

11. Composition pharmaceutique selon l'une quelconque des revendications 1 à 10, où un gel visqueux et/ou une dispersion colloïdale est formé quand la composition pharmaceutique est mise en contact avec un petit volume d'un solvant aqueux.

12. Composition pharmaceutique selon l'une quelconque des revendications 1 à 11, où le polymère soluble dans l'eau de faible masse moléculaire est un poly(oxyde d'éthylène) ayant une masse moléculaire de 100000 daltons et est présent en une quantité de 20 % à 50 % en poids de la composition pharmaceutique; le polyglycol est un copolymère d'éthylèneglycol et de propylèneglycol et est présent en une quantité de 10 % à 45 % en poids de la composition pharmaceutique; le polysaccharide est la gomme de xanthane et est présent en une quantité de 2 % à 6 % en poids de la composition pharmaceutique; le minéral de type argile est la bentonite sodique ou une combinaison de bentonite sodique et de talc et est présent en une quantité de 5 % à 25 % en poids de la composition pharmaceutique; et l'IPA est un opioïde choisi parmi l'oxycodone, l'oxymorphone, l'hydrocodone, l'hydromorphone, la codéine et la morphine.

13. Composition pharmaceutique selon la revendication 12, comprenant en outre le système effervescent facultatif comprenant a) un composant acide choisi parmi un acide organique, un acide inorganique, et leurs combinaisons et b) un composant base choisi parmi un bicarbonate de métal alcalin, un bicarbonate de métal alcalino-terreux, un carbonate de métal alcalin, un carbonate organique, et leurs combinaisons; et le système effervescent est présent en une quantité de 15 % à 50 % en poids de la composition pharmaceutique.

14. Procédé pour préparer une forme galénique solide orale de la composition pharmaceutique selon l'une quelconque des revendications 1 à 13, où le procédé comprend:
a) la formation d'un mélange comprenant le au moins un ingrédient pharmaceutique actif (IPA) ayant un potentiel pour les abus ou un sel pharmaceutiquement acceptable de celui-ci, le au moins un polymère soluble dans l'eau de faible masse moléculaire ayant une masse moléculaire de pas plus de 300000 daltons, le au moins un polyglycol, le au moins un polysaccharide, le au moins un minéral de type argile, et le système effervescent facultatif;
b) la mise du mélange sous forme d'une unité galénique solide; et
c) le chauffage de l'unité galénique solide pour donner la forme galénique solide orale.
